# EUROPEAN PATENT SPECIFICATION

(11) **EP 2 575 916 B1**
(45) Date of publication and mention of the grant of the patent: **04.04.2018**
(21) Application number: 11787407.3
(22) Date of filing: 26.05.2011
(51) Int. Cl.: A61L 31/04, A61L 31/14, A61F 13/00, A61L 24/04

(54) **ONE-PART MOISTURE-CURABLE TISSUE SEALANT**
EINTEILIGES FEUCHTIGKEITSHÄRTENDES GEWEBEVERSCHLUSSMITTEL
AGENT DE SCELLEMENT TISSULAIRE, À UN SEUL COMPOSANT, POUVANT DURCIR À L'HUMIDITÉ

(30) Priority: 30.09.2010 US 388321 P; 28.05.2010 US 349274 P
(43) Date of publication of application: 10.04.2013
(73) Proprietor: Cohera Medical, Inc., Pittsburgh, Pennsylvania 15212 (US); Beckman, Eric J., Aspinwall, Pennsylvania 15215 (US)
(72) Inventor: BECKMAN, Eric J., Aspinwall, Pennsylvania 15215 (US)
(74) Representative: Conroy, John
(86) International application number: PCT/US2011/038127
(87) International publication number: WO 2011/150199

(56) References cited:
- EP-A1- 2 111 877
- WO-A2-2005/003201
- US-A1- 2004 224 168
- US-A1- 2008 057 317
- US-A1- 2009 030 145
- US-A1- 2009 124 751
- US-A1- 2010 043 945

## Description

### TECHNICAL FIELD

This invention relates to moisture-curable sealants for sealing biological tissue.

### BACKGROUND

Tissue sealants are typically used to stop bleeding during vascular or liver surgery, eliminate air leaks in the lungs, and to prevent adhesions. Examples of sealants used for this purpose include fibrin products, polyethylene glycol products, and albumin-based products. In each case, the tissue sealant consists of two distinct components that are mixed together just prior to application to tissue to cause a rapid, irreversible chemical reaction. This reaction transforms the mixture from a low viscosity liquid into an elastic solid that coats the target tissue. The sealants are designed to degrade within a set period of time that typically ranges from days to weeks. One problem with such two-part sealants, however, is that the rapid cure times can cause the sealant applicator to clog.

EP-A-2 111 877 relates to a macromer composition for use as a tissue adhesive or sealant, which is biocompatible, non-immunogenic and biodegradable. The bioabsorbable macromer composition can be applied to living tissue and/or flesh of animals, including humans. The bioabsorbable macromer composition can be employed to adhere tissue edges, seal air/fluid leaks in tissues, adhere medical devices, i.e. implants, to tissue, and for tissue augmentation such as sealing or filling voids or defects in tissue.

WO-A-2005/003201 relates to alkoxysilane-terminated prepolymers and to compositions comprising prepolymers. WO-A-2005/003201 is based on the problem for not only providing blends of silane-terminated prepolymers but also to improve the silane-terminated prepolymers themselves with respect to the prior art. The improved prepolymers are not only to be distinguished by a high reactivity with respect to atmospheric humidity but show also an improved-tensile strength and, moreover, a considerably improved breaking elongation.

### SUMMARY

A tissue sealant is described that includes the reaction product of (a) a polyol having at least two groups capable of reacting with an alkoxy silane; and (b) an alkoxy silane having the formula: (R¹R²R³)-Si-CHz-Z where (i) Z is an -OH, -SH, -NCO, or NHR⁴ group, where R⁴ is hydrogen or an alkyl group; and (ii) each R¹, R², and R³, independently, is H, an alkoxy group, an alkyl group, a heteroalkyl group other than an alkoxy group, an aryl group, or a heteroaryl group, with the proviso that at least two of R¹, R², and R³ are alkoxy groups. The tissue sealant is moisture curable and biodegradable in a physiological environment.

As used herein, the term "alkyl" includes straight chain, branched, and cyclic alkyl groups.

The polyol may be an activated polyol. As used herein, the term "activated polyol" refers to a polyol in which one or more of the hydroxyl groups have been modified to create a molecule that is more reactive towards the alkoxy silane than the unmodified polyol.

In some embodiments, the activated polyol is the reaction product of a polyol and a polyfunctional linker molecule having at least one functional group capable of reacting with a hydroxyl group of the polyol, and at least one functional group capable of reacting with the Z group of the alkoxy silane. The functional group of the polyfunctional linker molecule that reacts with the hydroxyl group of the polyol can be different from, or the same as, the functional group of the polyfunctional linker molecule that reacts with the Z group of the alkoxy silane.

In some embodiments, the polyfunctional linker molecule is a polyisocyanate. Examples of suitable polyisocyanates include polyisocyanates derived from amino acids and amino acid derivatives such as lysine diisocyanate and derivatives thereof, lysine triisocyanate and derivatives thereof, and combinations thereof. Examples of suitable derivatives include alkyl esters (e.g., methyl and ethyl esters). Dipeptide derivatives can also be used. For example, lysine can be combined in a dipeptide with another amino acid (e.g., valine or glycine). Another representative example of a suitable polyfunctional linker molecule is maleic anhydride.

In some embodiments, the activated polyol includes an ester group capable of reacting with the alkoxy silane. The ester group may be created by reacting a hydroxyl group of a polyol with an anhydride to convert the hydroxyl group to a carboxylic acid group, and then reacting the carboxylic acid group with a reagent such as N-hydroxysuccinimide to create the ester group.

In some embodiments, the polyol is ionically charged. For example, the ionically charged polyol may include one or more sulfate, sulfonate, and/or ammonium ion functional groups.

In some embodiments, the reaction product of the polyol and alkoxy silane includes at least one hydrolyzable linkage. Examples of hydrolyzable linkages include esters, amides, urethanes, ureas, carbonates, and combinations thereof.

The alkoxy group of the alkoxysilane may be a C₁-C₆ alkoxy group, e.g., an ethoxy group. In some embodiments, two of R¹, R², and R³ are alkoxy groups, while in other embodiments each of R¹, R², and R³ is an alkoxy group. In some embodiments, the Z group of the alkoxysilane is an -NHR⁴ group.

In some embodiments, the polyol has a molecular weight that is no greater than 10,000, while in other embodiments it has a molecular weight that is no greater than 5,000. Representative examples include polyether polyols, polyester polyols, co-polyester polyether polyols, and combinations thereof. Two or more different polyols may be used in combination with each other and reacted with the alkoxysilane. As used herein, "different" means different molecular weights and/or chemical structures.

The tissue sealant can also include at least one reagent selected from the group consisting of solvents, diluents, catalysts, and combinations thereof. In use, the sealant is applied to a tissue surface, and cured in the presence of moisture associated with the tissue to seal the tissue surface. Because the sealant is a one-component composition (i.e. it includes one active molecule that moisture cures upon application to tissue), it is not necessary to mix two components prior to tissue application, thereby simplifying application from the user's perspective and avoiding the applicator clogging problems associated with two-component tissue sealants.

The details of one or more embodiments of the invention are set forth in the description below. Other features, objects, and advantages of the invention will be apparent from the description and from the claims.

### DETAILED DESCRIPTION

The tissue sealant includes the reaction product of a polyol and an alkoxy silane. The reaction product preferably includes at least one hydrolyzable linkage to promote biodegradability *in vivo.* Examples of hydrolyzable linkages include esters, amides, urethanes, ureas, carbonates, and combinations thereof.

The polyol may be an activated polyol in which one or more of the hydroxyl groups have been modified to create a molecule that is more reactive towards the alkoxy silane than the unmodified polyol. The activated polyol may be prepared by reacting a hydroxyl group of the polyol with a polyfunctional linker molecule having at least one functional group capable of reacting with a hydroxyl group of the polyol, and at least one functional group capable of reacting with the Z group of the alkoxy silane. Examples of suitable polyfunctional linker molecules are described in the Summary of the Invention, above. Alternatively, the activated polyol may be prepared by reacting a hydroxyl group of a polyol with an anhydride to convert the hydroxyl group to a carboxylic acid group, and then reacting the carboxylic acid group with a reagent such as N-hydroxysuccinimide to create an ester group. The ester group, in turn, is capable of reacting with the alkoxy silane.

Examples of suitable polyols are described in the Summary of the Invention, above. Specific examples of polyether polyols include polyethylene and polypropylene glycols. One or more of the hydroxyl groups may be activated. Specific examples of polyester polyols include polycaprolactone and polylactide diols. One or more of the hydroxyl groups may be activated.

The alkoxy silane has the formula: (R¹R²R³)-Si-CH₂-Z where (i) Z is an -OH, -SH, -NCO, or -NHR⁴ group. R⁴ is a hydrogen or an alkyl group (e.g., a C₁-C₆ alkyl group). Each R¹, R², and R³, independently, is H, an alkoxy group (e.g., a C₁-C₆ alkoxy group), an alkyl group (e.g., a C₁-C₆ alkyl group), a heteroalkyl group other than an alkoxy group (e.g., an alkyl amido or amido group), an aryl group (e.g., a phenyl group), or a heteroaryl group (e.g., a pyrrolyl, furyl, or pyridinyl group), with the proviso that at least two of R¹, R², and R³ are alkoxy groups. The alkyl groups may be straight chain, branched, or cyclic alkyl groups.

In one embodiment, the polyol is a polyalkylene glycol such as polyethylene glycol; the alkoxy silane is a trialkoxy silane such as a triethoxy silane in which the Z group is an alkylamino group such as a cyclohexylamino group (where R⁴ is a cyclohexyl group); and the polyfunctional linker molecule is a multi-functional isocyanate such as lysine diisocyanate ("LDI") or a derivative thereof (e.g., alkyl esters such as methyl or ethyl esters), or lysine triisocyanate ("LTI") or a derivative thereof (e.g., alkyl esters such as methyl or ethyl esters). The linker molecule reacts with the hydroxyl groups of the polyol to create an activated polyol.

In another embodiment, the polyol is a polyalkylene glycol such as polyethylene glycol in which one or more hydroxyl groups have been converted to activated ester groups, and the alkoxy silane is a trialkoxy silane such as a triethoxy silane in which the Z group is an alkylamino group such as a cyclohexylamino group (where R⁴ is a cyclohexyl group).

The sealants may further contain one or more reagents selected from the group consisting of solvents, diluents, catalysts, and combinations thereof. The reagents preferably are inert towards the polyol, trialkoxy silane, and polyfunctional linker molecule, and thus do not interfere with the reaction among these three reactants.

Examples of suitable catalysts, include tertiary amines (e.g., aliphatic tertiary amines) and organometallic compounds (e.g., bismuth salts and zirconium chelates). When the reactants include polyols and polyisocyanates, specific examples of useful catalysts include 1,4-diazabicyclo[2.2.2]octane ("DABCO"), 2,2'-dimorpholine diethyl ether ("DMDEE"), dibutyltin dilaurate ("DBTDL"), bismuth-2-ethylhexanoate, and combinations thereof.

The solvents and diluents may be used to modify the rheology of the sealant. Examples of suitable solvents include dimethylsulfoxide (DMSO), dimethylformamide (DMF), tetrahydrofuran (THF), glyme, and combinations thereof. Examples of suitable non-volatile diluents include dimethylsulfoxide (DMSO), propylene carbonate, diglyme, polyethylene glycol diacetates, polyethylene glycol dicarbonates, dimethylisosorbide, ethyl pyruvate, triacetin, triethylene glycol, and combinations thereof. Examples of suitable volatile diluents include hydrocarbons, perfluoroalkanes, hydrofluoroalkanes, carbon dioxide, and combinations thereof. A single reagent can perform multiple roles. Thus, for example, DMSO can function as both a solvent and a non-volatile diluent.

The sealants may also include one or more stabilizers. Examples include antioxidants (e.g., BHT and BHA), water scavengers (e.g., acyl and aryl halides, and anhydrides), Bronsted acids, and the like. Bronsted acids may also be used as catalysts.

The sealants may be prepared in either a single step reaction, in which reactants are combined together in a "single pot" reaction, or a multi-step reaction, in which the reactants are reacted sequentially. In either case, the reaction may be carried out in the presence of the aforementioned solvents, diluents, and/or stabilizers; alternatively, any or all of these reagents can be added after the reaction product has been created.

### Example 1

Polyethylene glycol having a molecular weight ranging from 300 to 1500 is dissolved in an inert diluent. The amount of the diluent typically represents 40-60% by weight of the total reaction mixture. Lysine ethyl ester diisocyanate ("LDI") is added at a 2:1 molar ratio relative to the glycol. A bismuth catalyst is added as well, and the glycol and diisocyanate are allowed to react with each other for several hours to create a polyurethane have isocyanate groups available for further reaction. Next, cyclohexylaminomethyl triethoxysilane is added at a 2:1 molar ratio relative to the glycol. The silane then reacts with the unreacted isocyanate groups to form the final moisture-curable product. Upon application to biological tissue, the product crosslinks in the presence of moisture associated with the tissue to form a smooth, elastomeric coating on the tissue that seals the tissue.

### Example 2

In a typical example, polyethylene glycol (molecular weight either 600, 1500, or 3400) is mixed with glutaric anhydride (2 moles anhydride per mole of polyethylene glycol); the mixture is heated to 70-80°C and allowed to stir overnight. IR shows the absence of anhydride peaks (1765, 1809 cm⁻¹) and the appearance of peaks representing the ester (1734 cm⁻¹) and carboxylic acid (1719 cm⁻¹). This intermediate is known as PEG-(COOH)₂.

PEG-(COOH)₂ is dissolved in anhydrous acetonitrile. N-hydroxy succinimide (2.2 moles per mole of PEG-(COOH)₂) is added and the mixture stirred until one phase is obtained. This solution is then cooled to < 10°C using an ice bath. At this point, dicylohexyl carbodiimide (2.2 moles per mole of PEG-(COOH)₂) in acetonitrile is added dropwise; after the addition is complete the mixture is stirred in the ice bath for 1 hour (a white precipitate begins to form). The ice bath is then removed and the mixture stirred at room temperature overnight. The precipitate (dicylohexyl urea) is removed by filtration. The filtrate is concentrated under vacuum, and the product precipitated into hexane. The product is redissolved in THF, filtered, then concentrated under vacuum. IR shows the characteristic NHS ester peaks at (1742, 1787, and 1815 cm⁻¹). This intermediate is known as PEG-(COONHS)₂.

PEG-(COOHNHS)₂ is dissolved in THF and heated to 50°C. At this point cyclohexylaminomethyl triethoxysilane (2 moles per mole of PEG-(COONHS)₂) is added and the mixture allowed to stir at 50°C for 24 hours. The solution is cooled, filtered, then the THF is removed under vacuum. IR shows the formation of the amide at 1678 cm⁻¹. A sealant is created by mixing this product with various amounts of ethyl pyruvate.

## Claims

1. A tissue sealant comprising the reaction product of:
(a) a polyol having at least two groups capable of reacting with an alkoxy silane; and
(b) an alkoxy silane having the formula: (R¹R²R³)-Si-CH2-Z where:
(i) Z is an -OH, -SH, -NCO, or -NHR⁴ group, where R⁴ is hydrogen or an alkyl group; and
(ii) each R1, R², and R³, independently, is H, an alkoxy group, an alkyl group, a heteroalkyl group other than an alkoxy group, an aryl group, or a heteroaryl group, with the proviso that at least two of R1, R², and R³ are alkoxy groups, wherein the tissue sealant is moisture-curable and biodegradable in a physiological environment.

2. A tissue sealant according to claim 1 wherein the reaction product comprises at least one hydrolysable linkage, in particular wherein the hydrolyzable linkage is selected from the group consisting of esters, amides, urethanes, ureas, carbonates, and combinations thereof.

3. A tissue sealant according to claim 1 wherein the alkoxy group is a C₁-C₆ alkoxy group or an ethoxy group.

4. A tissue sealant according to claim 1 wherein two of R1, R², and R³ are alkoxy groups or
wherein each of R1, R², and R³ is an alkoxy group, or
wherein Z is an -NHR⁴ group.

5. A tissue sealant according to claim 1 wherein the polyol has a molecular weight that is no greater than 10,000 or no greater than 5,000.

6. A tissue sealant according to claim 1 wherein the polyol is selected from the group consisting of polyether polyols, polyester polyols, co-polyester polyether polyols, and combinations thereof.

7. A tissue sealant according to claim 1 wherein the polyol is an activated polyol.

8. A tissue sealant according to claim 7 wherein the activated polyol is prepared by reacting one or more hydroxyl groups with a polyfunctional linker molecule having at least one functional group capable of reacting with the hydroxyl groups of the polyol, and at least one functional group capable of reacting with the Z group of the alkoxy silane.

9. A tissue sealant according to claim 8 wherein the functional group of the polyfunctional linker molecule that reacts with the hydroxyl group of the
polyol to form the activated polyol is different from the functional group of the polyfunctional linker molecule that reacts with the Z group of the alkoxy silane, or
wherein the functional group of the polyfunctional linker molecule that reacts with the hydroxyl group of the polyol to form the activated polyol is the same as the functional group of the polyfunctional linker molecule that reacts with the Z group of the alkoxy silane.

10. A tissue sealant according to claim 8, wherein the polyfunctional linker molecule comprises maleic anhydride or
a polyisocyanate, in particular
wherein the polyisocyanate is selected from the group consisting of lysine diisocyanate and derivatives thereof, lysine triisocyanate and derivatives thereof, and combinations thereof.

11. A tissue sealant according to claim 7 wherein the activated polyol comprises one or more ester groups, in particular wherein the activated polyol is prepared by converting one or more hydroxyl groups of a polyol to carboxylic acid groups, and esterifying the carboxylic acid groups to create an activated polyol comprising one or more ester groups.

12. A tissue sealant according to claim 1 wherein the tissue sealant comprises the reaction product of the alkoxy silane and at least two different polyols.

13. A tissue sealant according to claim 1 wherein the polyol is ionically charged inparticular
wherein the polyol includes at least one functional group selected from the group consisting of sulfates, sulfonate, ammonium ions, and combinations thereof.

14. A tissue sealant according to claim 1, wherein the tissue sealant further comprises at least one reagent selected from the group consisting of solvents, diluents, catalysts, and combinations thereof.

15. A tissue sealant according to one of claims 1 to 14 for use in the treatment of bleedings.

## Patentansprüche

1. Gewebeversiegelungsmittel, umfassend das Reaktionsprodukt von:
(a) einem Polyol mit mindestens zwei Gruppen, die mit einem Alkoxysilan reagieren können; und
(b) einem Alkoxysilan mit der Formel: (R¹R²R³)-Si-CH2-Z, wobei:
(i) Z für eine -OH-, -SH-, -NCO- oder -NHR⁴-Gruppe steht, wobei R⁴ für Wasserstoff oder eine Alkylgruppe steht; und
(ii) R¹, R² und R³ jeweils unabhängig für H, eine Alkoxygruppe, eine Alkylgruppe, eine Heteroalkylgruppe, die von einer Alkoxygruppe verschieden ist, eine Arylgruppe oder eine Heteroarylgruppe stehen, mit der Maßgabe, dass mindestens zwei der Variablen R¹, R² und R³ für Alkoxygruppen stehen, wobei das Gewebeversiegelungsmittel feuchtigkeitshärtbar und in einer physiologischen Umgebung biologisch abbaubar ist.

2. Gewebeversiegelungsmittel nach Anspruch 1, wobei das Reaktionsprodukt mindestens eine hydrolysierbare Bindung umfasst, insbesondere wobei die hydrolysierbare Bindung aus der Gruppe bestehend aus Estern, Amiden, Urethanen, Harnstoffen, Carbonaten und Kombinationen davon ausgewählt ist.

3. Gewebeversiegelungsmittel nach Anspruch 1, wobei es sich bei der Alkoxygruppe um eine C₁-C₆-Alkoxygruppe oder eine Ethoxygruppe handelt.

4. Gewebeversiegelungsmittel nach Anspruch 1, wobei zwei der Variablen R¹, R² und R³ für Alkoxygruppen stehen oder
wobei R¹, R² und R³ jeweils für eine Alkoxygruppe stehen oder
wobei Z für eine -NHR⁴-Gruppe steht.

5. Gewebeversiegelungsmittel nach Anspruch 1, wobei das Polyol ein Molekulargewicht von höchstens 10.000 oder höchstens 5000 aufweist.

6. Gewebeversiegelungsmittel nach Anspruch 1, wobei das Polyol aus der Gruppe bestehend aus Polyetherpolyolen, Polyesterpolyolen, Copolyesterpolyetherpolyolen und Kombinationen davon ausgewählt ist.

7. Gewebeversiegelungsmittel nach Anspruch 1, wobei es sich bei dem Polyol um ein aktiviertes Polyol handelt.

8. Gewebeversiegelungsmittel nach Anspruch 7, wobei das aktivierte Polyol durch Umsetzen einer oder mehrerer Hydroxylgruppen mit einem polyfunktionellen Linkermolekül mit mindestens einer funktionellen Gruppe, die mit den Hydroxylgruppen des Polyols reagieren kann, und mindestens einer funktionellen Gruppe, die mit der Z-Gruppe des Alkoxysilans reagieren kann, hergestellt wird.

9. Gewebeversiegelungsmittel nach Anspruch 8, wobei die funktionelle Gruppe des polyfunktionellen Linkermoleküls, die mit der Hydroxylgruppe des Polyols unter Bildung des aktivierten Polyols reagiert, von der funktionellen Gruppe des polyfunktionellen Linkermoleküls, die mit der Z-Gruppe des Alkoxysilans reagiert, verschieden ist oder wobei die funktionelle Gruppe des polyfunktionellen Linkermoleküls, die mit der Hydroxylgruppe des Polyols unter Bildung des aktivierten Polyols reagiert, mit der funktionellen Gruppe des polyfunktionellen Linkermoleküls, die mit der Z-Gruppe des Alkoxysilans reagiert, identisch ist.

10. Gewebeversiegelungsmittel nach Anspruch 8, wobei das polyfunktionelle Linkermolekül Maleinsäureanhydrid oder ein Polyisocyanat umfasst, insbesondere wobei das Polyisocyanat aus der Gruppe bestehend aus Lysindiisocyanat und Derivaten davon, Lysintriisocyanat und Derivaten davon und Kombinationen davon ausgewählt ist.

11. Gewebeversiegelungsmittel nach Anspruch 7, bei dem das aktivierte Polyol eine oder mehrere Estergruppen umfasst, insbesondere wobei das aktivierte Polyol durch Umwandeln einer oder mehrerer Hydroxylgruppen eines Polyols in Carbonsäuregruppen und Verestern der Carbonsäuregruppen zur Bildung eines aktivierten Polyols mit einer oder mehreren Estergruppen hergestellt wird.

12. Gewebeversiegelungsmittel nach Anspruch 1, wobei das Gewebeversiegelungsmittel das Reaktionsprodukt von dem Alkoxysilan und mindestens zwei verschiedenen Polyolen umfasst.

13. Gewebeversiegelungsmittel nach Anspruch 1, wobei das Polyol ionisch geladen ist, insbesondere wobei das Polyol mindestens eine funktionelle Gruppe aus der Gruppe bestehend aus Sulfaten, Sulfonat, Ammoniumionen und Kombinationen davon enthält.

14. Gewebeversiegelungsmittel nach Anspruch 1, wobei das Gewebeversiegelungsmittel ferner mindestens ein Reagenz aus der Gruppe bestehend aus Lösungsmitteln, Verdünnungsmitteln, Katalysatoren und Kombinationen davon umfasst.

15. Gewebeversiegelungsmittel nach einem der Ansprüche 1 bis 14 zur Verwendung bei der Behandlung von Blutungen.

## Revendications

1. Agent de scellement tissulaire comprenant le produit réactionnel de :
(a) un polyol comportant au moins deux groupes capables de réagir avec un alcoxysilane ; et
(b) un alcoxysilane ayant la formule : (R¹R²R³)-Si-CH₂-Z, dans laquelle :
(i) Z est un groupe -OH, -SH, -NCO, or -NHR⁴, dans lequel R⁴ est un atome d'hydrogène ou un groupe alkyle ; et
(ii) chaque groupe R¹, R² et R³, indépendamment, est un atome d'hydrogène, un groupe alcoxy, un groupe alkyle, un groupe hétéroalkyle autre qu'un groupe alcoxy, un groupe aryle, ou un groupe hétéroaryle, sous réserve qu'au moins deux des groupes R¹, R² et R³ soient des groupes alcoxy, l'agent de scellement tissulaire étant durcissable à l'humidité et biodégradable dans un environnement physiologique.

2. Agent de scellement tissulaire selon la revendication 1, dans lequel le produit réactionnel comprend au moins une liaison hydrolysable, en particulier dans lequel la liaison hydrolysable est sélectionnée dans le groupe constitué d'esters, d'amides, d'uréthanes, d'urées, de carbonates et de combinaisons de ceux-ci.

3. Agent de scellement tissulaire selon la revendication 1, dans lequel le groupe alcoxy est un groupe alcoxy C₁-C₆ ou un groupe éthoxy.

4. Agent de scellement tissulaire selon la revendication 1, dans lequel deux des groupes R¹, R² et R³ sont des groupes alcoxy, ou
dans lequel chacun des groupes R¹, R² et R³ est un groupe alcoxy, ou
dans lequel Z est un groupe -NHR⁴.

5. Agent de scellement tissulaire selon la revendication 1, dans lequel le polyol a une masse moléculaire qui n'est pas supérieure à 10 000 ou qui n'est pas supérieure à 5 000.

6. Agent de scellement tissulaire selon la revendication 1, dans lequel le polyol est sélectionné dans le groupe constitué de polyéthers polyols, de polyesters polyols, de co-polyéthers-polyesters polyols, et de combinaisons de ceux-ci.

7. Agent de scellement tissulaire selon la revendication 1, dans lequel le polyol est un polyol activé.

8. Agent de scellement tissulaire selon la revendication 7, dans lequel le polyol activé est préparé en faisant réagir un ou plusieurs groupes hydroxyle avec une molécule de liaison polyfonctionnelle comportant au moins un groupe fonctionnel capable de réagir avec les groupes hydroxyle du polyol, et au moins un groupe fonctionnel capable de réagir avec le groupe Z de l'alcoxysilane.

9. Agent de scellement tissulaire selon la revendication 8, dans lequel le groupe fonctionnel de la molécule de liaison polyfonctionnelle qui réagit avec le groupe hydroxyle du polyol pour former le polyol activé est différent du groupe fonctionnel de la molécule de liaison polyfonctionnelle qui réagit avec le groupe Z de l'alcoxysilane, ou
dans lequel le groupe fonctionnel de la molécule de liaison polyfonctionnelle qui réagit avec le groupe hydroxyle du polyol pour former le polyol activé est le même que le groupe fonctionnel de la molécule de liaison polyfonctionnelle qui réagit avec le groupe Z de l'alcoxysilane.

10. Agent de scellement tissulaire selon la revendication 8, dans lequel la molécule de liaison polyfonctionnelle comprend l'anhydride maléique ou
un polyisocyanate, en particulier
dans lequel le polyisocyanate est sélectionné dans le groupe constitué du diisocyanate de lysine et de ses dérivés, du triisocyanate de lysine et de ses dérivés, et de combinaisons de ceux-ci.

11. Agent de scellement tissulaire selon la revendication 7, dans lequel le polyol activé comprend un ou plusieurs groupes ester, en particulier
dans lequel le polyol activé est préparé en convertissant un ou plusieurs groupes hydroxyle d'un polyol en groupes acide carboxylique, et en estérifiant les groupes acide carboxylique pour créer un polyol activé comprenant un ou plusieurs groupes ester.

12. Agent de scellement tissulaire selon la revendication 1, l'agent de scellement tissulaire comprenant le produit réactionnel de l'alcoxysilane et d'au moins deux différents polyols.

13. Agent de scellement tissulaire selon la revendication 1, dans lequel le polyol est chargé ioniquement, en particulier
dans lequel le polyol comprend au moins un groupe fonctionnel sélectionné dans le groupe constitué de sulfates, de sulfonates, d'ions ammonium, et de combinaisons de ceux-ci.

14. Agent de scellement tissulaire selon la revendication 1, l'agent de scellement tissulaire comprenant en outre au moins un réactif sélectionné dans le groupe constitué de solvants, de diluants, de catalyseurs, et de combinaisons de ceux-ci.

15. Agent de scellement tissulaire selon l'une des revendications 1 à 14, destiné à une utilisation dans le traitement de saignements.
